# EUROPEAN PATENT APPLICATION

(11) **EP 0 572 131 A1**
(43) Date of publication of application: **01.12.1993**
(21) Application number: 93303568.5
(22) Date of filing: 07.05.1993
(51) Int. Cl.: A61B 17/39

(54) **Surgical scissors with bipolar coagulation feature**

(30) Priority: 21.05.1992 US 887212; 05.02.1993 US 13844
(71) Applicant: Everest Medical Corporation, Minneapolis, Minnesota 55441 (US)
(72) Inventor: Rydell, Mark A., Golden Valley, Minnesota 55434 (US)
(74) Representative: MacGregor, Gordon

(57) **Abstract**

A surgical scissors (10) for use in endoscopic surgery allows both mechanical cutting and RF coagulation. The scissors blades (60, 62) are mounted on the distal end of an elongated rigid tube (12) dimensioned to fit through a working lumen of an endoscope or a trocar. The blades (60, 62) are a composite, including a metal blank and a ceramic layer (92, 104) adhered to the opposed blade faces. A scissors style handle (24) is coupled via a longitudinally rigid rod member (66) to the movable blade (62), causing it to pivot relative to the fixed blade (60) when the scissors-type handle (24) is manipulated. The blades (60, 62) are electrically insulated from each other along their entire lengths.

## Description

### BACKGROUND OF THE INVENTION

### I. Field of the Invention:

This invention relates generally to the design of a surgical scissors, and more particularly to a surgical scissors incorporating bipolar electrodes as its blade elements, such that mechanical cutting with subsequent electrocoagulation can be achieved without requiring an instrument exchange.

### II. Discussion of the Prior Art:

Electrocoagulating instruments include at least one conductive electrode. Radio frequency energy is conducted through this electrode to either a remote conductive body-plate (monopolar) or to a second, closely-spaced conductive electrode (bipolar). Current passing through the gap between the two electrodes will coagulate blood and other body fluids placed between them.

Monopolar electrocautery instruments suffer from the fact that the return path between the active electrode and the large area body-plate can be unpredictable as the electrical current seeks the return electrode through the path of least resistance. With bipolar electrosurgical instruments, however, because the two electrodes are closely spaced to one another, usually at the distal end of an instrument handle, the return path is very short and only involves the tissue and fluids in the short path between the electrodes.

There is available in the prior art a scissors-type instrument for mechanically snipping tissue during the course of an endoscopic procedure. Such a scissors comprises of pair of blades fabricated from metal and disposed at the distal end of an elongated tubular member whose outside diameter is sufficiently small to allow it to be passed through the working lumen of an endoscope, a laparoscope or other similar devices known in the art. Disposed at the proximal end of the rigid tube is a scissors-type handle having a pair of members, each with a finger-receiving loop therein which are pivotally coupled to one another. An appropriate linkage is made between the handle members and the blades so that manipulation of the handle members will result in an opening and closing of the blades relative to one another. When using a mechanical cutting scissors of this type to excise tissue, when a blood vessel is cut, bleeding results. At that point, it is generally necessary for the surgeon to remove the scissors instrument from the working lumen of the endoscope and then insert an electrocoagulator down the endoscope to the site of the bleeder. This instrument exchange is time-consuming and in a surgical procedure where moments count, it would be desirable to have a scissors-type instrument for cutting but which also incorporates the ability to coagulate blood and other body tissue using RF energy.

There is also available in the prior art monopolar scissors where both of the scissors blades form one pole and with a body plate being the second pole. To date, however, there is not available in the marketplace a bipolar electrosurgical scissors where its two blades are electrically isolated from one another and comprise the bipolar electrode pair. With metal-to-metal contact along the sharpened edges of the two blades, an electrical short results. Furthermore, the attempt to use a rivet or screw as the pivot point for the blades is another area where short-circuiting is likely to occur. When such a short exists, the electrical current does not flow through the blood or body tissue to effect coagulation, but instead, follows the short-circuit path from one electrode to the other.

It is accordingly a principal object of the present invention to provide a bipolar, electrocoagulating instrument having scissors blades for the mechanical cutting of tissue.

Another object of the present invention is to provide bipolar scissors having a miniaturized distal blade configuration that allows the instrument to be inserted through a laparoscope, trocar or the working lumen of an endoscope.

Still another object of the present invention is to provide a bipolar-type scissors instrument which utilizes a push-rod-and-pivot combination to cause movement of the scissors' blade through manipulation of a scissors-style handle mechanism at the proximal end of the instrument and wherein the scissors blades may be simultaneously energized from a RF source to effect the electrocoagulation of cut tissue.

### SUMMARY OF THE INVENTION

The foregoing objects and advantages of the invention are achieved by providing a bipolar scissors for insertion into a laparoscope, trocar or endoscope for effecting electrocoagulation of blood and tissue during laparoscopic or other endoscopic surgery. The scissors blades at the distal tip of the instrument perform cutting of the tissue by mechanical shearing action. The two blades are effectively insulated from one another along their entire lengths, allowing them to function as bipolar electrodes for electrocoagulating small blood vessels in the surgical field.

The instrument includes a scissors-type handle having first and second pivoting members, each with a finger-receiving loop on one end of each and extending from the opposite end of one is an elongated, rigid tubular member of a size capable of being inserted through the trocar or endoscope. Affixed to the distal end of the rigid tubular member is a first blade composite which comprises a metal blank having a suitable ceramic layer bonded to one major surface thereof, the ceramic being honed to define a sharp cutting edge. Pivotally joined to the first blade by an insulating pivot member is a second blade composite, also having a metal blank with a ceramic substrate bonded to one major surface thereof. When the two blade blanks are pivotally joined together, the ceramic layers are in face-to-face relationship and because the cutting edges thereof are honed, the blades are capable of cutting tissue when made to move in a scissors-like manner with tissue placed between the cutting edges thereof.

Extending through the lumen of the elongated tubular member is a wire or rod which is rigid in the longitudinal direction and which is coupled at its proximal end to one of the handle members and at its other end by an electrically insulated articulating linkage to one of the scissors blades. By appropriately manipulating the handle members, a snipping action of the blades results.

The instrument further includes means for applying an RF voltage across the gap between the two metal blade blanks which are maintained separated from one another by the ceramic faces bonded to these blanks. As such, the blades of the instrument itself can be used as a bipolar electrocoagulation device, obviating the need for doing an instrument exchange when it is necessary to coagulate blood and tissue following the mechanical cutting thereof.

### DESCRIPTION OF THE DRAWINGS

The foregoing features, objects and advantages of the invention will become apparent to those skilled in the art from the following detailed description of a preferred embodiment, especially when considered in conjunction with the accompanying drawings in which like numerals in the several views refer to corresponding parts.
Figure 1 is a side elevation view of the preferred embodiment, the drawing being partially sectioned to better illustrate the working elements of the embodiment;
Figure 2 is a cross-section taken along the lines 2-2 in Figure 1;
Figure 3a is a partial plan view of the instrument of Figure 1;
Figure 3b is an enlarged perspective view of a linkage cover;
Figure 4 is an enlarged view of the cutting blade portion of the instrument of Figure 1;
Figure 5 is a cross-sectional view taken along the lines 5-5 in Figure 4;
Figure 6 is a cross-sectional view like that of Figure 5 but in accordance with an alternative embodiment;
Figure 7 is a side view of the movable blade of the instrument of Figure 1; and
Figure 8 is a top view of the movable blade illustrated in Figure 7.

### DESCRIPTION OF THE PREFERRED EMBODIMENT

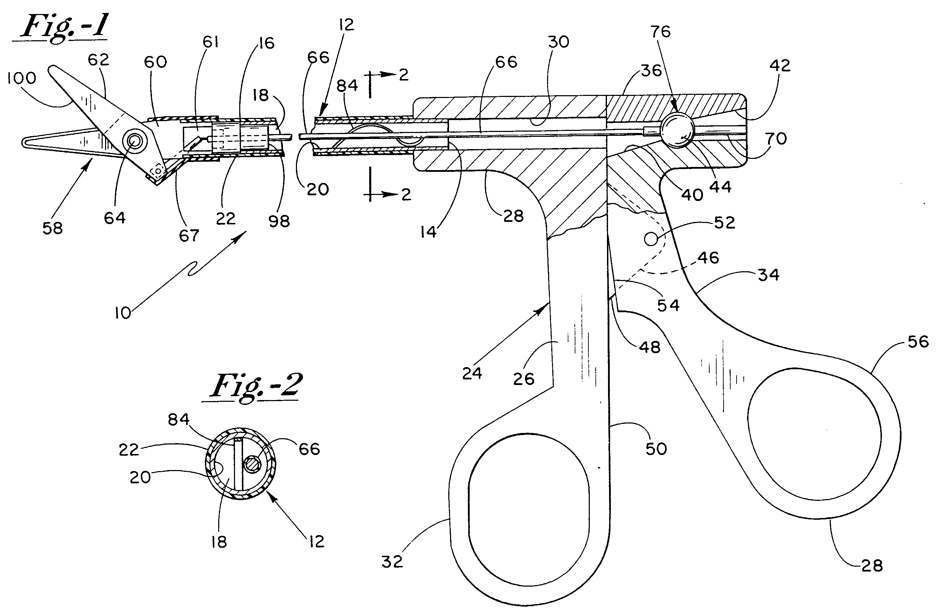

Referring first to Figure 1, there is indicated generally by numeral 10 a bipolar electrosurgical scissors for use in endoscopic procedures. As used herein, the term "endoscope" or "endoscopic" is used in its general sense and is meant to include laparoscopes, cystoscopes, and other related devices where minimally invasive surgery is carried out by utilizing a viewing device having a working lumen for receiving another surgical instrument therethrough. It includes an elongated tubular member 12 of a diameter and length sufficient to pass through another instrument and to reach the surgical field where cutting and excision of tissue and other organs is to take place. The tubular member 12 has a proximal end 14, a distal end 16 and a lumen 18 running therebetween. As can best be seen in the cross-sectional view of Figure 2, the elongated tubular member 12 comprises a metal tube 20 coated with an insulating layer 22. The insulating coating 22 is preferably a polymer, such as Teflon®, which provides excellent electrically insulating properties while yielding a lubricous surface, allowing it to more readily slide through the lumen of an endoscope or a trocar.

Affixed to the proximal end 14 of the tube 12 is a scissors-type handle indicated generally by numeral 24. It includes a first handle member 26 having first and second ends, the first end including a tubular barrel 28 having a bore 30 extending therethrough into which is fitted and bonded the proximal end 14 of the tubular member 12. Formed on the second end of the first handle member 26 is a finger loop 32 intended to receive the forefinger of the surgeon's hand.

The handle assembly 24 further includes a second handle member 34 having a first end 36 and a second end 38, the first end including a pair of longitudinal bores 40 and 42, each with a sloping side wall leading to a transversely extending bore 44. The second handle member 34 includes a longitudinally extending triangular slot 46 for receiving a triangular-shaped ear 48 which is integrally molded with and projects outward from a flat edge surface 50 of the first handle member 26. A pivot pin, as at 52, secures the handle member 34 to the handle member 26. The front edge surface of the second handle member 34 is relieved as at 54. Hence, when the surgeon's thumb is introduced through the finger loop 56 and with the forefinger through the finger loop 32, the first and second handle members 26 and 34 can be squeezed toward one another, causing the end 36 of the second handle member to pull away from the edge 50 of the barrel portion 28 of the first handle member.

Press-fit into the distal end 16 of the rigid tubular member 12 is a blade assembly indicated generally by numeral 58. As will be explained with greater particularity hereinbelow, the blade assembly 58 may comprise a fixed blade 60 and a movable blade 62 pivotally joined to one another by a pin or screw 64 which extends through bores formed through the two blade members. Alternatively, both blades can be movable.

With reference to Figures 1 and 2 of the drawings, it can be seen that there is extending through the lumen 18 of the tubular member 12 a longitudinally rigid wire or rod 66 which is preferably covered with a layer of insulation so that it cannot become shorted to the inside wall of the tubular member 12. The rod 66 is connected by an articulated coupler, as at 68, to the movable blade member 62 at a location displaced from the pivot point 64 of the two blades. A cover 61 covers the linkage site.

The proximal end of the rigid rod 66 extends longitudinally outward from the proximal end of the tube 12 and completely through the bore 30 of the first handle member 26 and through the tapered bore 40 in the second handle member 34 where it enters a conductive crimp tube 70. One of the two leads 72 and 74 leading to the electrosurgical generator is also fitted into the crimp tube 70. The crimp tube 70 passes through a diametric bore formed in a cylinder bearing 76 which is loosely inserted within the transverse bore 44 so that when the handle 34 is moved reciprocally in a scissors-type movement relative to the fixed handle member 26 about the pivot point 52, the cylinder 76 is free to rotate slightly as a bearing, thus avoid flexure or bending of the rod 66 and/or crimp tube 70.

The other of the lead wires 72 and 74 also has the uninsulated end thereof inserted into a second crimp tube 78 which extends parallel to the crimp tube 70 and passes through a bore formed diametrically in the cylinder bearing 76. Fitted into the other end of the crimp tube 78 is a further conductor 80. With reference to the top view of Figure 3a during manufacture, when a crimping tool is allowed to enter the elongated slot 82 formed in the cylinder 76, each of the crimp tubes can be compressed to tightly engage the ends of the wires 72 and 74 and the conductive wires or rods 66 and 80 making a good electrical and mechanical junction therebetween. The wire or rod 80 is also rigid in the longitudinal direction and connects at its distal end to the proximal end of a copper wave spring 84 which extends for a predetermined distance through the lumen 18 of the tube 12 to establish sliding electrical contact between the wave spring and the inner metal wall of the tube defining the lumen 18.

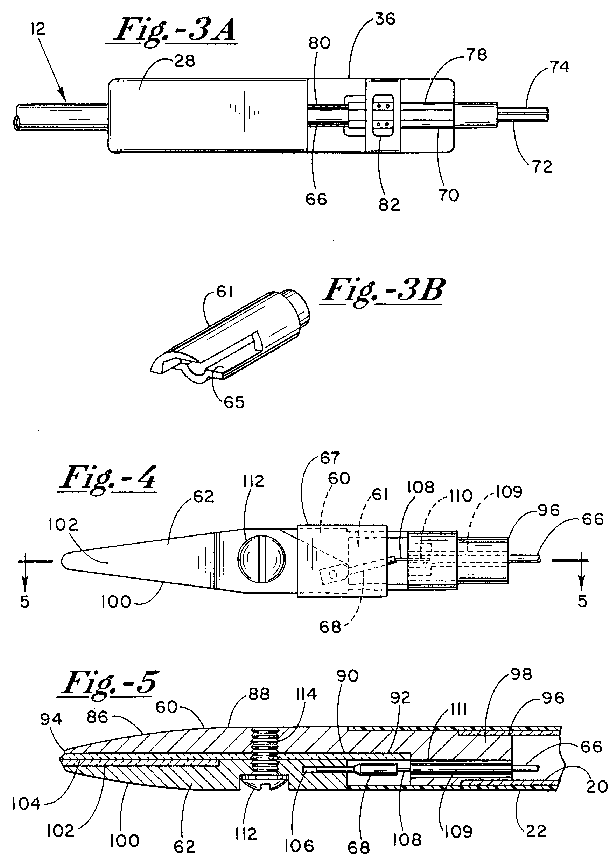

Figure 3b illustrates the linkage cover 61 alone which is disposed to bridge the articulating linkage comprising the coupler 68 and cable segment 108 as shown in Figures 1 and 4. As is evident, the cover 61 has a cut-out portion 65 which accommodates the proximal portion of the movable blade 62 when the scissors 10 is operated. The purpose of the cover 61, preferably constructed of high temperature plastic or other appropriate insulation material, is to electrically isolate the linkage to the movable blade 62 from the stationary blade 60 to thereby avoid the possibility of a conductive fluid path between the two.

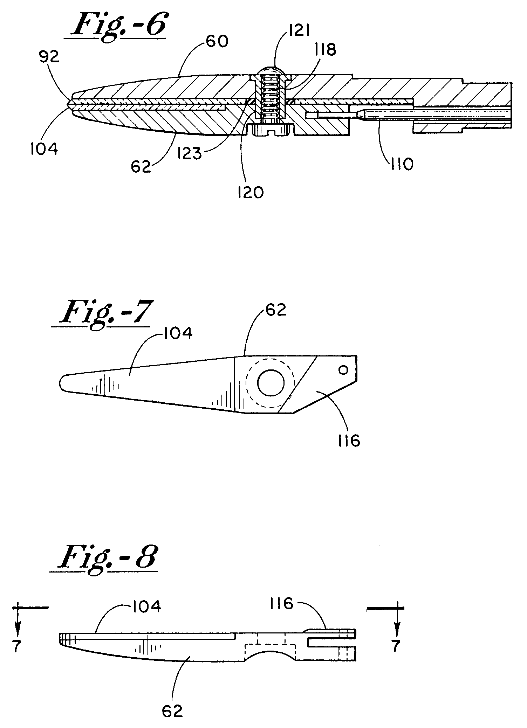

To better understand the constructional features of the blade assembly 58, reference is made to the views of Figures 4 and 5 which show the blades closed relative to one another. It is to be noted that the instrument shown in Figure 4 includes an insulating boot 67, described later. The fixed blade 60 includes a metal blank 86 having an arcuate profile defining its outer major surface 88 and a flat inner surface 90. Bonded to this flat surface 90 is a ceramic sheet or layer 92. The distal end 94 of the conductive metal blank 86 is rounded as can be seen in the side elevation view of Figure 4 while its proximal end 96 includes an integrally formed cylindrical stub portion 98 which is dimensioned to cooperate with the internal diameter of the tube 12 to create a friction-fit and to firmly hold the blade assembly 58 in place at the distal end of the tube 12 while conducting electrical current from the tube to the fixed blade. The blank 86 is preferably formed from stainless steel and the ceramic surface 92 may be aluminum oxide or zirconia ceramic.

The movable blade 62 also comprises a metal (stainless steel) blank having a slightly rounded outer peripheral surface 100 and an inner flat surface 102 supporting a ceramic layer 104 thereon. The working edge 106 of the ceramic layer 104 is beveled to a sharp edge. In practice, it has been found that if the ceramic layers 92 and 104 are each approximately 0.020 in. in thickness and with their working edges beveled at an angle of 45°, an appropriate gap approximately 0.040 in. wide is created between the metal blanks comprising the fixed blade and the movable blade when the blades are closed relative to one another. An RF current traversing the blood and/or body tissue bridging this gap can cauterize tissue and coagulate blood and other body fluids.

The movable blade 62 is provided with a slot as at 106 (Figure 5) for receiving one end of the articulated coupler 68 therein. As can best be seen in Figure 4, the articulated coupler 68 includes a flat section received in the slot 106 and a length of metal tubing into which is fitted a short length of flexible cable 108. Once the cable is inserted into the length of tubing, the tubing is crimped to tightly hold the cable 108 therein. Likewise, the other end of the cable segment 108 is fitted into a crimp tube 110 which also receives the elongated ridge member 66 therein. An insulating tube 109 is inserted in the bore 111 formed through the cylindrical stub 96 to surround the crimp tube 110 and insulate it from contact with the fixed blade. With the flattened end of the articulated coupler 68 inserted into the slot 106 and pinned in place, and with a span of only approximately 0.050 in. between the end of the coupler 68 and the crimp tube 110, back and forth movement of the rigid rod in the axial direction will cause the movable blade to move between an open and closed position with no kinking or buckling of the flexible link afforded by the cable segment 108. If needed, an elastic insulating boot 67, preferably constructed of rubber, can cover the proximal portion of the blades so that, when the movable blade 62 is moved, its resultantly projecting proximal corner cannot inadvertently cause current flow to adjacent tissue.

As shown in Figure 5, a screw 112, formed from a nonconductive material such as, for example, ceramic or high temperature plastic, passes through an aperture formed in the movable blade 62 and into a threaded bore 114 formed in the fixed blade blank 86. Once the desired clamping force between the mating surfaces of the two blades has been established by appropriately tightening the screw 112, it may be held in place by swaging or otherwise treating the screw so that it cannot loosen itself.

To maintain the sharpened beveled edges of the ceramic layers 92 and 104 in intimate contact during the snipping movement of the blades and to avoid the necessity of having the blades bent or hollow ground to achieve this result, it has been found expedient to include an integrally formed ramp on one or the other of the movable and fixed blades. With reference to Figures 7 and 8, the ramp is illustrated as being integrally formed on the movable blade 62. In particular, near the proximal end portion of the movable blade on its inner major surface there is formed a sloping ramp surface 116. This is achieved by appropriately machining the blank so as to leave a rise of approximately 0.010 in. When the movable blade 62 is affixed to the stationary blade 60 by the screw 112, the ceramic surface 104 thereof, and especially its working edge is biased by the ramp to intersect with the line of the working edge of the fixed blade 60. Hence, when the handle members 26 and 34 are manipulated, the point of contact between the working edges of the fixed and movable blades will move along the length of the blade as it opens and closes, creating the desired shearing action of tissue positioned between the blades.

The view of Figure 6 is substantially identical to that of Figure 5, except that, instead of using a non-conductive ceramic screw to join the blade halves in pivoting relationship relative to one another, in the view of Figure 6, a conductive screw 118 is employed. To insure that the conductive screw will not create a short circuit between the metal blanks comprising the fixed and movable blades, an insulating plastic sleeve 120 encircled by an epoxy bushing seal ring 123 is fitted into the bore of the fixed blade 60 to thereby isolate the screw 118 from ohmic contact with the metal blade blank. A drop of epoxy 121 is applied at the end of the screw 118 for insulation of the screw end. This forms a fluid tight seal at the pivot necessary to prevent shorting in the presence of conductive fluids such as a saline solution. As would be apparent to a skilled artisan, a rivet could be employed instead of a screw in joining the blade halves in pivoting relationship to each other.

Various modifications of the invention, as described herein, will become apparently to those skilled in the art from the reading of the present specification and an examination of the drawings. For example, rather than having one blade fixed and the other movable, it is also possible to run two rigid rods through the lumen of the tubular member and have those rigid rods appropriately coupled to two blade members which are pivotally joined one to the other in such a way that both blade members are movable relative to one another. Moreover, instead of using a short length of cable as at 108 in Figures 4 and 5, it is also possible to utilize a rigid link which is pivoted at opposed ends thereof to the movable blade member and to a member joined to the rigid rod. Thus, when the rigid rod is moved reciprocally back and forth appropriate manipulation of the handle grip, the movable blade will be made to open and close in a snipping action. The choice of the preferred arrangement shown in Figures 4 and 5 is based upon ease of manufacturing considerations. That is to say, it is much simpler to crimp a short length of cable into two slightly spaced lengths of tubing than it is to assemble a tiny linkage to a blade and to a coupler on the end of the push wire using pins to achieve a pivot joint.

It is believed that the operation of the present invention is fully apparent from the description of the constructional features of the instrument. By using the thumb and forefinger to spread and close the handle members relative to one another, the push rod 66 is moved back and forth within the lumen of the tube and acts to pivot the movable blade relative to the fixed blade. Because of the ceramic interfaces between the fixed and movable blades, the metal portions thereof are maintained at a predetermined gap suitable for electrocautery and electrocoagulation. Moreover, the ramp formed on the face of one of the fixed and movable blades causes the beveled edge of the ceramic layers to contact one another at a point with that point moving along the length of the blade as it is manipulated between its open and closed position. The pivot, if otherwise conductive, is made non-conductive and sealed to prevent shorting in fluid. The manner in which the RF energy is applied to the blades has already been described, and need not be repeated here.

This invention has been described herein in considerable detail in order to comply with the Patent Statutes and to provide those skilled in the art with the information needed to apply the novel principles and to construct and use such specialized components as are required. However, it is to be understood that the invention can be carried out by specifically different equipment and devices, and that various modifications, both as to the equipment details and operating procedures, can be accomplished without departing from the scope of the invention itself.

## Claims

1. A surgical instrument for mechanically shearing tissue and for cauterizing tissue and coagulating blood and other body fluids, comprising:
(a) an elongated tubular member having a proximal end, a distal end and a lumen extending therebetween;
(b) first and second scissors blades pivotally joined and electrically insulated from one another, along their entire lengths, said scissors blades being affixed to said distal end of said tubular member;
(c) means affixed to said proximal end of said tubular member for pivoting one of said first and second scissors blades relative to the other through a linkage means having a distal portion connected to the pivoting blade; and
(d) means for applying a radio frequency voltage across said first and second scissors blades.

2. The surgical instrument as in Claim 1 having in addition electrical insulation means disposed to insulate said distal portion of the linkage means.

3. The surgical instrument as in Claim 1 or 2 wherein said first and second scissors blades are a composite formed from stainless steel and a ceramic insulator.

4. The surgical instrument as in Claim 3 wherein said first and second scissors blades each include a metal blank having a ceramic layer affixed thereto, said ceramic layer on said first scissors blade being juxtaposed in face-to-face relation to said ceramic layer on said second scissors blade when said scissors blades are pivotally joined to thereby create a predetermined insulating gap between said metal blanks.

5. The surgical instrument as in Claim 4 wherein each of said ceramic layers has a beveled cutting edge.

6. The surgical instrument as in Claim 4 wherein said first and second scissors blades are pivotally joined by an insulating member.

7. The surgical instrument as in Claim 6 wherein said insulating member is a ceramic screw.

8. The surgical instrument as in Claim 6 wherein said insulating member includes an insulating sleeve and a bushing seal surrounding a metal screw.

9. The surgical instrument as in Claim 3 wherein said ceramic insulator is a member of the group including aluminum oxide and zirconia.

10. The surgical instrument as in Claim 5 wherein the metal blank of one of said first and second scissors blades includes a ramp surface on a predetermined area thereof, said ramp surface maintaining said beveled cutting edge of the ceramic layer on said first blade in rubbing contact with said beveled cutting edge of the ceramic layer on said second blade when said first and second blades are pivoted relative to one another.

11. A surgical instrument for mechanically shearing tissue and for cauterizing tissue and coagulating blood and other body fluids, comprising:
(a) an elongated tubular member having a proximal end, a distal end and a lumen extending therebetween;
(b) first and second scissors blades pivotally joined and electrically insulated from one another, along their entire lengths, said scissors blades being affixed to said distal end of said tubular member;
(c) means affixed to said proximal end of said tubular member for pivoting one of said first and second scissors blades relative to the other, said means comprising:
(i) a first handle member having first and second ends, said first end being affixed to said proximal end of said tubular member and said second end terminating in a finger grip;
(ii) a second handle member having first and second ends, said first end including a finger grip, said first handle member being pivotally joined to said second handle member at a location between first and second ends of each;
(iii) an elongated, longitudinally rigid rod having a proximal end, a distal end and extending through said lumen of said tubular member, said proximal end of said rigid rod being coupled to said second end of said second handle member; and
(iv) electrically insulated means for coupling said distal end of said rigid rod to one of said first and second blades; and
(d) means for applying a radio frequency voltage across said first and second scissors blades.

12. The surgical instrument as in Claim 11 wherein said means for coupling said distal end of said rigid rod to one of said first and second blades comprises an articulating linkage.

13. The surgical instrument as in Claim 11 wherein said elongated tubular member is electrically conductive and coated on its exterior surface with an electrically insulating polymer.

14. The surgical instrument as in Claim 13 wherein said means for applying an RF voltage across said first and second scissors blades includes:
(a) a first electrical lead adapted to be coupled to an electrosurgical generator and connected to said rigid rod at said proximal end thereof; and
(b) a second electrical lead adapted to be coupled to an electrosurgical generator and to said tubular member.

15. The surgical instrument as in Claim 13 wherein said second electrical lead is coupled to said tubular member by an axially movable conductive member.

16. The surgical instrument as in Claim 15 wherein said axially movable conductive member is a wave spring disposed within said lumen of said tubular member and engaging said tubular member at a plurality of points along the length thereof.

17. The surgical instrument as in Claim 12 wherein the outside diameter of said elongated tubular member and the maximum transverse dimension of said first and second scissors blades when in a closed position are generally equal and of a size permitting them to be passed through the working lumen of an endoscope or trocar.

18. The surgical instrument as claimed in Claim 12 wherein the articulating linkage is covered with an insulating cover to thereby provide electrical insulation to the linkage.

19. The surgical instrument as claimed in Claim 18 wherein a proximal portion of each of the blades is covered by an electrically insulated elastic boot.
